# EUROPEAN PATENT APPLICATION

(11) **EP 4 052 749 A1**
(43) Date of publication of application: **07.09.2022**
(21) Application number: 22156781.1
(22) Date of filing: 15.02.2022
(51) Int. Cl.: A61M 16/00, A61M 16/10, A61M 16/12, B01F 25/312, B01F 23/10, B01F 23/20, F04F 5/14, F04F 5/46

(54) **MIXTURE ENTRAINMENT DEVICE**

(30) Priority: 05.03.2021 US 202117249596
(71) Applicant: Honeywell International Inc., Charlotte, NC 28202 (US)
(72) Inventor: HARRISON, Alex, Charlotte, 28202 (US); PLUMMER, David Alan, Charlotte, 28202 (US)
(74) Representative: Haseltine Lake Kempner LLP

(57) **Abstract**

An example injector includes a first conduit defining a first flow path including a first choke for a first fluid and a second conduit defining a second flow path including a second choke for a second fluid. The second choke is defined by a converging region upstream and a diverging region downstream of the second choke. The example injector further includes a mixing region after both the diverging region of the second flow path and the first choke and configured to receive the first fluid the second fluid, and an outlet configured to allow the first fluid and the second fluid to exit the mixing region. The first and second chokes are configured to allow a constant mass flow of the first and second fluids, respectively, to flow into the mixing region independent of a pressure at the outlet.

## Description

### TECHNICAL FIELD

The present disclosure relates to injectors.

### BACKGROUND

An injector is a system of ducting and nozzles used to direct the flow of a high-pressure fluid in such a way that a lower pressure fluid is entrained in the jet and carried through a duct to a region of higher pressure. An injector consists of a body filled with a secondary fluid, into which a motive fluid is injected. The motive fluid induces the secondary fluid to move. Injectors exist in many variations, and can have several stages, each repeating the same basic operating principle, to increase their overall effect.

### SUMMARY

In one example, this disclosure describes an injector includes a first conduit defining a first flow path for a first fluid, wherein the first flow path includes a first choke; a second conduit defining a second flow path for a second fluid, wherein the second flow path includes a second choke, wherein the second choke is defined by a converging region upstream of the second choke and a diverging region downstream of the second choke; a mixing region configured to receive the first fluid from the first flow path and the second fluid from the second flow path, wherein the mixing region is after the diverging region of the second flow path and after the first choke; and an outlet configured to allow the first fluid and the second fluid to exit the mixing region, wherein the first choke is configured to allow a constant mass flow of the first fluid to flow into the mixing region independent of a pressure at the outlet, wherein the second choke is configured to allow a constant mass flow of the second fluid to flow into the mixing region independent of the pressure at the outlet.

In another example, this disclosure describes a method of mixing a first fluid and a second fluid includes choking, via a first choke in a first flow path defined by a first conduit, the flow of a first fluid; choking, via a second choke in a second flow path defined by a second conduit, the flow of a second fluid, wherein the second choke is defined by a converging region upstream of the second choke and a diverging region downstream of the second choke; mixing the first fluid and the second fluid in a mixing region, wherein the mixing region is after the first choke of the first flow path and after the diverging region of the second flow path wherein the first choke is configured to allow a constant mass flow of the first fluid to flow into the mixing region independent of a pressure at the outlet, wherein the second choke is configured to allow a constant mass flow of the second fluid to flow into the mixing region for independent of the pressure at the outlet.

In another example, this disclosure describes a jet pump includes a pump; and an injector includes a first conduit defining a first flow path of a first fluid including a first choke; a second conduit defining a second flow path of a second fluid including a second choke, wherein the second choke is defined by a converging region upstream of the second choke and a diverging region downstream of the second choke; a mixing region configured to receive the first fluid from the first flow path and the second fluid from the second flow path, wherein the mixing region is after the diverging region of the second flow path and after the first choke; and an outlet configured to allow the first fluid and the second fluid to exit the mixing region, wherein the first choke is configured to allow a constant mass flow of the first fluid to flow into the mixing region independent of a pressure at the outlet, wherein the second choke is configured to allow a constant mass flow of the second fluid to flow into the mixing region for independent of the pressure at the outlet.

The details of one or more examples are set forth in the accompanying drawings and the description below. Other features, objects, and advantages will be apparent from the description and drawings, and from the claims.

### BRIEF DESCRIPTION OF THE DRAWINGS

The details of one or more examples are set forth in the accompanying drawings and the description below. Other features, objects, and advantages will be apparent from the description and drawings, and from the claims.
FIG. 1 is a conceptual diagram of an example injector, in accordance with the techniques of this disclosure.
FIG. 2 is a conceptual diagram of an example injector, in accordance with the techniques of this disclosure.
FIG. 3 is a conceptual diagram of a ventilator including an example injector, in accordance with the techniques of this disclosure.
FIG. 4 is a conceptual diagram of a ventilator including an example injector, in accordance with the techniques of this disclosure.
FIG. 5 is a conceptual diagram of an example system including an example injector, in accordance with the techniques of this disclosure.
FIG. 6 is a flowchart of an example method of mixing two fluids, in accordance with examples of the present disclosure.

### DETAILED DESCRIPTION

Conventional pneumatic jet pump designs include an injector including only a single a choke that is located in the primary fluid flow path before the fluid mixing region, e.g., a venturi in the flow path of the primary fluid before the mixing region. Pressure variation at the outlet of the mixing region may then cause a variation in the amount of secondary fluid drawn into the mixing region, e.g., a variation in the mass flow of the secondary fluid into the mixing region. Consequently, the mixing ratio between the primary and secondary fluids in the mixing region varies with the varying mass flow of the secondary fluid caused by varying pressure conditions downstream of the mixing region.

Many applications, such as medical ventilators, could potentially benefit from a substantially constant mixing ratio of a primary and secondary fluid when the pressure downstream of the mixing region is not constant.

In accordance with one or more aspects of this disclosure, an injector comprises a first flow path of a primary fluid (alternatively referred to as the motive and/or drive fluid) including a first choke (e.g., a first venturi), a second flow path of a secondary fluid (alternatively referred to as ) including a second choke (e.g., a second venturi), and a mixing region configured to receive the primary fluid and the secondary fluid after both the first and second chokes. Both the first and second chokes may be configured such that the mass flow of the primary and secondary fluids into the mixing region is independent of a pressure at an outlet of the mixing region, e.g., to allow a constant mass flow of the primary and secondary fluids in the mixing region independent of pressure variation at the outlet of the mixing region, and thereby allow a constant mixing ratio of the primary and secondary fluids in the mixing region independent of variation at the outlet of the mixing region. In some examples, both the first and second chokes may be configured such that the speed of the primary and secondary fluids in the first and second chokes, respectively, are at least the speed of sound. In some examples, single and/or multiple convergent-divergent sections in the secondary flow paths, e.g., single or multiple venturis in the secondary flow path may be configured to choke the flow of the secondary fluid at low pressure ratios across the inlet to the mixing region from the secondary flow path.

An injector in accordance with one or more aspects of this disclosure may improve the stability of the mixing ratio of the injector, simplify flow measurement, improve the accuracy of control of the mixing of the primary and secondary fluids across wide range of outlet pressures by controlling upstream pressure, minimize the size of the injector, e.g., one or both of the length and/or diameter of the primary or secondary flow paths, and reduce the complexity and cost of the injector. For example, choking both the primary and secondary flow paths before the mixing region reduces variation in the mass flow of each of the primary and secondary fluids into the mixing region over a wide range of downstream pressure conditions, which reduces variation in the mixing ratio of the primary and secondary fluids independent of downstream pressure conditions. Establishing choking conditions in both the primary and secondary flow paths before the mixing region in which the speed of the primary and secondary fluids in the respective chokes is at least the speed of sound enables a substantially constant mass flow of each of the primary and secondary fluids into the mixing region over a wide range of downstream pressure conditions, which enables a substantially constant in the mixing ratio of the primary and secondary fluids independent of downstream pressure conditions. In some examples, such an injector may enable choking of the secondary fluid path via multiple chokes which may enable the length of the secondary flow path may be shortened for a given mass flow of the secondary fluid into the mixing region. In some examples, such an injector may enable measurement of the flow of the secondary and/or primary fluids to be measured and/or controlled by varying the pressure upstream of the nozzle of the primary flow path, which may simplify measurement and control of the mixture of primary and secondary fluids in the presence of varying pressure conditions downstream of the mixing region.

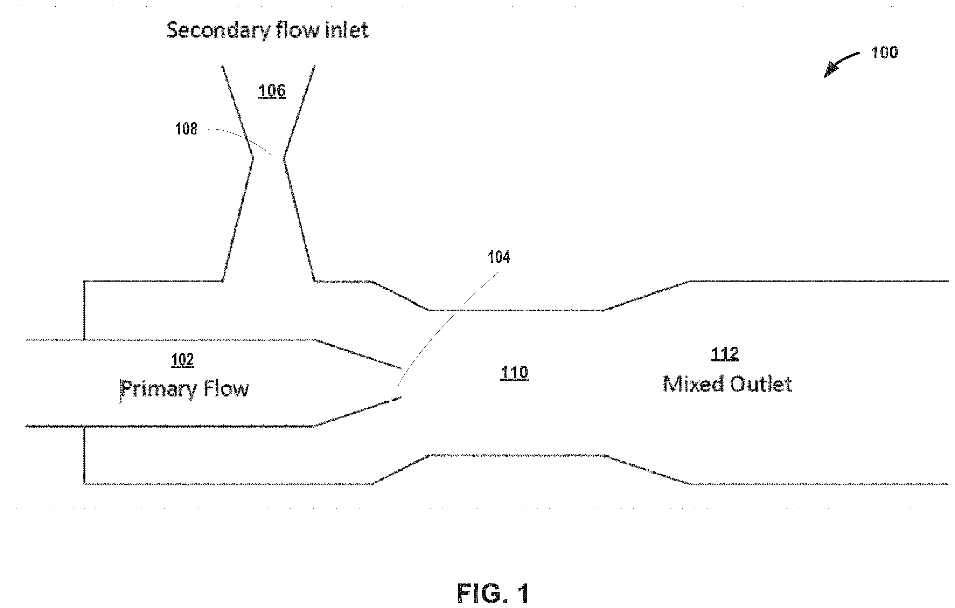

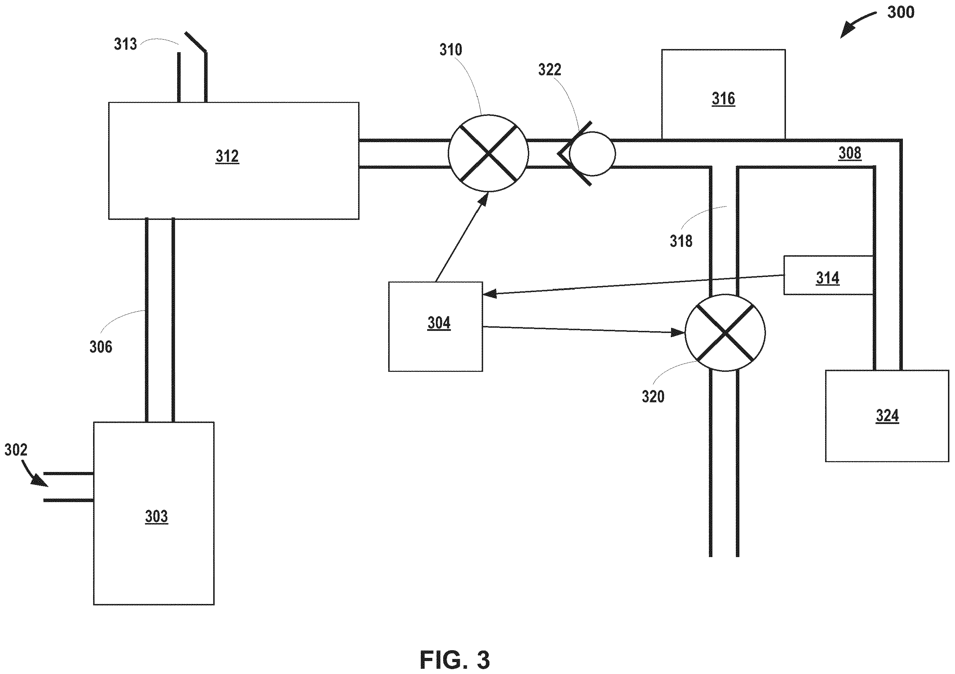

FIG. 1 is a conceptual diagram of an example injector 100, in accordance with the techniques of this disclosure. Injector 100 includes primary flow path 102, primary choke 104, secondary flow path 106, secondary choke 108, mixing region 110, and outlet 112. Injector 100 may be an example of injectors 312 and/or 412 of FIGS. 3-4. Although injector 100 and its operation is described in the context of a supply gas as a primary fluid and ambient air as a secondary fluid, injector 100 may be used with any suitable primary fluid and secondary fluid such as gases and/or gas/liquid mixtures.

Primary flow path 102 may be fluidically connected to a primary fluid supply, e.g., a primary gas supplied via a pneumatic connection such as connection 302 and/or 402 of FIGS. 3-4, which are further described below. The primary gas may be supplied within primary flow path 102 at a pressure higher than a pressure after primary choke 104. Primary choke 104 may be a venturi and may be configured to increase the speed of the primary gas within primary choke 104. In some examples, primary choke 104 may be configured to increase the speed of the primary gas to at least the speed of sound, e.g., such that the primary gas is at a sonic condition in primary choke 104. In the example shown, choke 104 is illustrated as a nozzle including a converging region upstream of a throat and/or minimum cross-sectional area of primary choke 104, e.g., the nozzle outlet, and without a diverging region downstream of the throat. In some examples, primary choke 104 may be defined by a converging region upstream of a throat of primary choke 104 and a diverging region downstream from the throat of primary choke 104. For example, primary choke 104 may be formed of primary flow path 102 walls which may form a converging region upstream of the throat of primary choke 104 and, in some examples, a diverging region downstream of the throat of primary choke 104. Generally, the converging (and diverging) region of primary choke 104 may have any suitable shape. In some examples, one or both of a converging and diverging region of primary choke 104 may have a conical shape. Primary choke 104 may be formed from any suitable material, e.g., plastic, metal, glass, or any suitable material.

Secondary flow path 106 may be fluidically connected to a secondary gas supply (e.g., or fluid supply), such as ambient air via an inlet such as air inlet 313 and/or 413 of FIGS. 3-4, which are further described below. Secondary choke 108 may be a venturi and may be configured to increase the speed of the secondary gas. In some examples, secondary choke 108 may be configured to increase the speed of the secondary gas to at least the speed of sound, e.g., such that the gas is at a sonic condition in secondary choke 108. In some examples, secondary choke 108 may be defined by a converging region upstream of the secondary choke 108, e.g., a throat and/or minimum cross-sectional area of secondary choke 108, and a diverging region downstream from the secondary choke 108. For example, secondary choke 108 may be formed of secondary flow path 106 walls which may form a converging region upstream of the throat of secondary choke 108 and a diverging region downstream of the throat of secondary choke 108. Generally, the converging and diverging regions of secondary choke 108 may have any suitable shape. In some examples, one or both of the converging and diverging regions of secondary choke 108 may have a conical shape. Secondary choke 108 may be formed from any suitable material, e.g., plastic, metal, glass, or any suitable material.

Mixing region 110 is fluidically connected to the outlets of primary flow path 102 and secondary flow path 106 and downstream from primary choke 104 and secondary choke 108. Mixing region 110 may receive the primary gas from primary flow path 102 and secondary gas from secondary flow path 106 downstream of primary choke 104 and secondary choke 108. In some examples, injector 100 may be or may be a portion of a jet pump, e.g., the primary gas flowing from primary flow path 102 may provide a motive force inducing the secondary gas flowing from the secondary flow path 106 to move. For example, a jet pump may include a pump, such as a centrifugal pump, a positive-displacement pump, an axial-flow pump, and the like, to move the primary gas. In some examples, the primary gas may be oxygen and the secondary gas may be ambient air.

In some examples, choking both the primary flow path 102 and the secondary flow path 106 via primary choke 104 and secondary choke 108 may enable a constant mass flow to be maintained through both primary flow path 102 and the secondary flow path 106 over a wide range of downstream pressure conditions, e.g., the mass flow of the primary and secondary gases may be independent of downstream pressures, such as the pressure in mixing region 108, outlet 112, or a pressure at any other point downstream from, and fluidically connected to, injector 100. In some examples, the constant mass, e.g., mass flow substantially independent of downstream pressures, of the primary and secondary gases may allow a constant mixing ratio (e.g., substantially independent of downstream pressures) of the primary and secondary gases. In some examples, choking both the primary flow path 102 and the secondary flow path 106 may provide both a constant mix ratio of the primary gas and the secondary gas and provide a constant total mixed flow, e.g., flow of the mixed primary and secondary gases, over a wide range of outlet 112 pressures. In some examples, choking both the primary flow path 102 and the secondary flow path 106 may allow for the flow of the mixed gases to be measured and/or controlled by varying the pressure upstream of primary choke 104, e.g., by controlling the pressure of the supply gas in primary flow path 102.

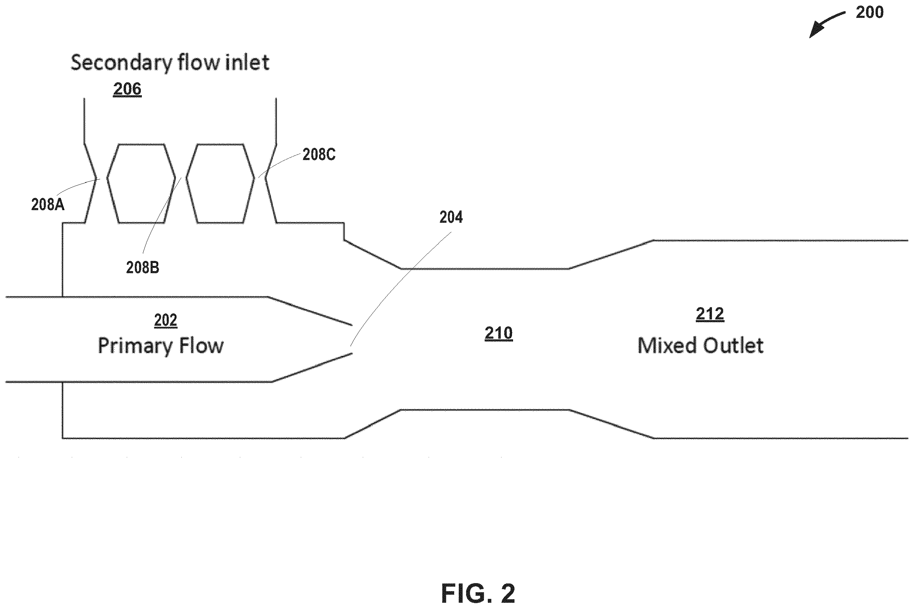

FIG. 2 is a conceptual diagram of an example injector 200, in accordance with the techniques of this disclosure. Injector 200 includes primary flow path 202, primary choke 204, secondary flow path 206, secondary chokes 208A, 208B, and 208C, mixing region 210, and outlet 212. Injector 200 may be an example of injectors 312 and/or 412 of FIGS. 3-4 and may be substantially similar to injector 100 except that instead of a single secondary choke, injector 200 includes a plurality of secondary chokes 208A-208C. Although injector 200 and its operation is described in the context of a supply gas as a primary fluid and ambient air as a secondary fluid, injector 200 may be used with any suitable primary fluid and secondary fluid such as gases and/or gas/liquid mixtures.

Primary flow path 202 and secondary flow path 206 may be substantially similar to primary flow path 102 and secondary flow path 106 of FIG. 1 described above. Similarly, primary choke 204, mixing region 210, and outlet 212 may be substantially similar to primary choke 104, mixing region 110, and outlet 112 of FIG. 1 described above.

In the example shown, injector 200 includes a plurality of secondary chokes 208A, 208B, and 208C. Each of secondary chokes 208A, 208B, and 208C may be substantially similar to secondary choke 108 of FIG. 1 described above. Although the example shown includes three secondary chokes, injector 200 may include fewer (e.g., two), or more secondary chokes. In some examples, the length, width, and overall size of each of secondary chokes 208A, 208B, and 208C may be reduced relative to secondary choke 108, and the length of secondary flow path 206 may be reduced relative to secondary flow path 106, while still providing the same and/or increased mass flow, relative to injector 100, of the secondary gas to mixing region 210. In the example shown, secondary chokes 208A, 208B, and 208C are in parallel, e.g., the secondary gas may flow through any single secondary choke 208A, 208B, or 208C, but not more than one of secondary chokes 208A, 208B, and 208C, into mixing region 210. In some examples, one or more of secondary chokes 208A, 208B, and 208C may be sequential, e.g., such that the secondary gas may flow through one or more of secondary chokes 208A, 208B, and 208C into mixing region 210. In some examples, injector 200 may include a plurality of secondary chokes in parallel and in sequence in any suitable order.

FIG. 3 is a conceptual diagram of a ventilator 300 including an example injector 312, in accordance with the techniques of this disclosure. Ventilator 300 includes pneumatic connection 302, balanced demand valve (BDV) 303, electronic control circuitry 304, supply path 306, inspiratory path 308, inspiratory valve 310 (Valve I 310 in FIG. 3), injector 312, pressure sensor 314, dump valve 316, expiratory path 318, expiratory valve 320 (Valve E 320 in FIG. 3), non-return valve 322, and output 324. Although injector 312 and its operation is described in the context of ventilator 300 and supply gas, ambient air, and oxygen gas, injector 312 may be used independently or with any suitable system and may be used with other fluids such as other gases and/or gas/liquid mixtures.

A user, such as a doctor, nurse, or other medical clinician, may connect ventilator 300 to a fluid supply, e.g., a gas supply, via a pneumatic connection 302. Pneumatic connection 302 may, for example, include conduit, piping and/or tubing configured to define a pathway for gas from the gas supply into ventilator 300. Pneumatic connection 302 may, for example, be configured to allow the pressurised supply gas to communicate without leaking. Pneumatic connection 302 may include a connector and an internal passage to allow the gas to flow to BDV 303.

Electronic control circuitry 304 may include, or be coupled to, a user interface device that allows a user of ventilator 300 to set operating parameters for ventilator 300. Electronic control circuitry 304 may, for example, be a variable-frequency-variable-duty-cycle electronic control circuit that forms the basis of the control of ventilator 300. Electronic control circuitry 304 can provide the timing framework which is used to deliver conditioned gas flow to the lungs of a patient.

Ventilator 300 is configured to deliver conditioned gas to the lungs of a patient via supply path 306 and inspiratory path 308. Electronic control circuitry 304 is configured to control the opening and closing of inspiratory valve 310 at a specific frequency to regulate the amount of conditioned gas being supplied to the patient and to control a "breaths per minute" being supplied to the patient (e.g., the human lungs of the patient) via inspiratory path 308. Generally speaking, a longer open interval for inspiratory valve 310 results in ventilator 300 delivering to a patient a larger volume of conditioned gasper breath, and more open intervals (e.g., more open intervals per minute) results in ventilator 300 delivering more breaths per minute. Electronic control circuitry 304 may alter the amount of conditioned gas being supplied and the number of breaths per minute being supplied by ventilator 300 by varying the open-close pattern of inspiratory valve 310. The volume of conditioned gas being driven into the patient may be selected by a user and regulated by electronic control circuitry 304.

Electronic control circuitry 304 may be configured to open inspiratory valve 310 for a period of time controlled by timing circuitry of electronic control circuitry 304 to achieve a desired inspiration volume. The time for which inspiratory valve 310 is held open may depend on the pressure of the gas supply, e.g., connected to pneumatic connection 302.

Supply path 306 and air inlet 313 may be fluidically connected to injector 312. Injector 312 may include at least one choke, e.g., at least one venturi, supplied by supply path 306, and at least one choke and/or venturi fluidically connected to a secondary flow path supplied by air inlet 313. Air inlet 313 is configured to be opened and closed to allow ambient air to mix with the supply gas within injector 312. Generally, mixing ambient air with the supply gas produces a conditioned gas with a reduced oxygen concentration relative to the supply gas. In some examples, supply path 306 and the secondary flow path supplied by air inlet 313 may include conduit, piping and/or tubing configured to define a pathway for a fluid, e.g., the supply gas and the secondary gas such as ambient air, respectively. Supply path 306 and the secondary flow path may be configured to allow a pressurized gas to communicate without leaking and may include one or more connectors and internal passage(s) to allow a gas to flow within the internal passage(s).

Injector 312 includes a mixing region fluidically connected to both supply path 306 and the secondary flow path after the chokes in both supply path 306 and the secondary path. In some examples, the supply gas flows at a speed that is at least the speed of sound within the supply path choke and may provide motive force for drawing in ambient air via air inlet 313 and the secondary flow path. In some examples, the ambient air may flow at a speed that is at least the speed of sound within the secondary path choke. In some examples, choking both the supply path and the secondary path enables the mass flow of both the supply gas and the ambient air to be insensitive to downstream pressure, e.g., pressure variation in inspiratory path 308. In some examples, injector 312 may provide a mixing ratio that is insensitive to downstream pressure by virtue of providing mass flows of the supply gas and the ambient air that are insensitive to downstream pressure variations.

In some examples, as used herein, "upstream" and "downstream" refer to a position relative to a flow of a fluid or gas. For example, a gas flowing within ventilator 300 flows through injector 312 before flowing through inspiratory path 308, and thus the pressure in inspiratory path 308 is downstream of the mass flows of the supply path and the secondary path within injector 312. Additionally, as illustrated and described above with respect to FIG. 1, secondary choke 108 may be defined by a converging region and a diverging region (e.g., converging and diverging conduit, piping, tubing, and the like) that form a throat and/or minimum cross-sectional area, e.g., secondary choke 108. A gas may flow through secondary choke from the converging region to the diverging region, and thus the converging region is referred to as upstream of secondary choke 108 and the diverging region is referred to as downstream of secondary choke 108.

In some examples, injector 312 may provide a choked flow of mixed supply gas and ambient air via nozzles that are sufficiently small, such that a pressure ratio across injector 312 may be approximately 2 to 1, or 1.5 to 1, or 1.25 to 1, or any suitable pressure ratio, e.g., between the pressure in supply path 306 and a pressure in inspiratory path 308. With supply path 306 producing a choked flow, the output rate (e.g., volume per second) of inspiratory path 308 may be determined based on the pressure in supply path 306, which can be sensed by pressure sensor 314, and may stay constant even if a pressure downstream from injector 312 changes. By using a choked flow through both supply path 306 and the secondary ambient air flow path to limit a flow into inspiratory path 308, electronic control circuitry 304 can determine the output rate of conditioned gas being delivered to inspiratory path 308 regardless of a pressure downstream from inspiratory path 308, e.g., at output 324, which may be the patient's lungs.

From the output rate, electronic control circuitry 304 can determine a volume of conditioned gas being delivered to a patient for a respiratory cycle. To increase the volume of conditioned gas for a future respiratory cycle, electronic control circuitry 304 can increase the amount of time which inspiratory valve 310 remains open. To decrease the volume of conditioned gas for a future respiratory cycle, electronic control circuitry can decrease the amount of time which inspiratory valve 310 remains open.

Inspiratory path 308 includes pressure sensor 314 which is configured to determine a pressure in inspiratory path 308. Pressure sensor 314 can have any suitable configuration, such as, but not limited to, a pressure transducer, a pressure gauge, or the like. Pressure sensor 314 can be configured to generate an output indicative of any suitable pressure measurement, such as, but not limited to, an absolute pressure and/or a differential pressure.

A user of ventilator 300 may set a maximum respiratory pressure that is appropriate for a particular patient. During an inspiration period, pressure sensor 314 may determine a pressure in inspiratory path 308, and if a user adjustable pre-defined peak pressure target is reached, then electronic control circuitry 304 may cause inspiratory valve 310 to close, thus holding the pressure for the remainder of the inspiration time. Each time the peak pressure setting is hit, curtailing the inspired volume of conditioned gas from being delivered, ventilator 300 may produce a visible or audible warning, such as a flashing light or beeping, thus giving the user feedback that the desired volume of conditioned gas is not being reached.

Ventilator 300 also includes dump valve 316, which provides a mechanical backup pressure reduction mechanism in the event that the operation of pressure sensor 316, electronic control circuitry 304, and inspiratory valve 310 does not adequately reduce the pressure in inspiratory path 308. Dump valve 316 may, for example, be a spring valve configured to open at a specific pressure. In some examples, the specific pressure may be substantially near 15.75 inches of water gauge (inWG), but other valves that open at different pressures may also be used.

During expiration, air is released from the lungs of the patient and out of ventilator 300 via path 318 by opening expiratory valve 320. Electronic control circuitry 304 may synchronize the opening and closing of inspiratory valve 310 with the opening and closing of expiratory valve 320, such that both are not simultaneously open. A positive end-expiratory pressure (PEEP) may represent a desired residual pressure in the lung and may be specified by a user of ventilator 300 (and input to control circuitry 304 using any suitable mechanism). Electronic control circuitry 304 may cause expiratory valve 320 to close when the pressure has decayed to a PEEP setting. Electronic control circuitry 304 may cause expiratory valve 320 to open multiple times to bring the pressure to the desired PEEP level. If the PEEP level is reached before the end of an expiratory period, then electronic control circuitry 304 may hold expiratory valve 320 closed for the remaining time of the expiratory period.

In some examples, inspiratory path 308 and expiratory path 318 may include conduit, piping and/or tubing configured to define a pathway for a fluid, e.g., the mixed gas from injector 312 and air released from the lungs of the patient, respectively. Inspiratory path 308 and expiratory path 318 may be configured to allow a pressurized gas to communicate without leaking and may include one or more connectors and internal passage(s) to allow a gas to flow within the internal passage(s).

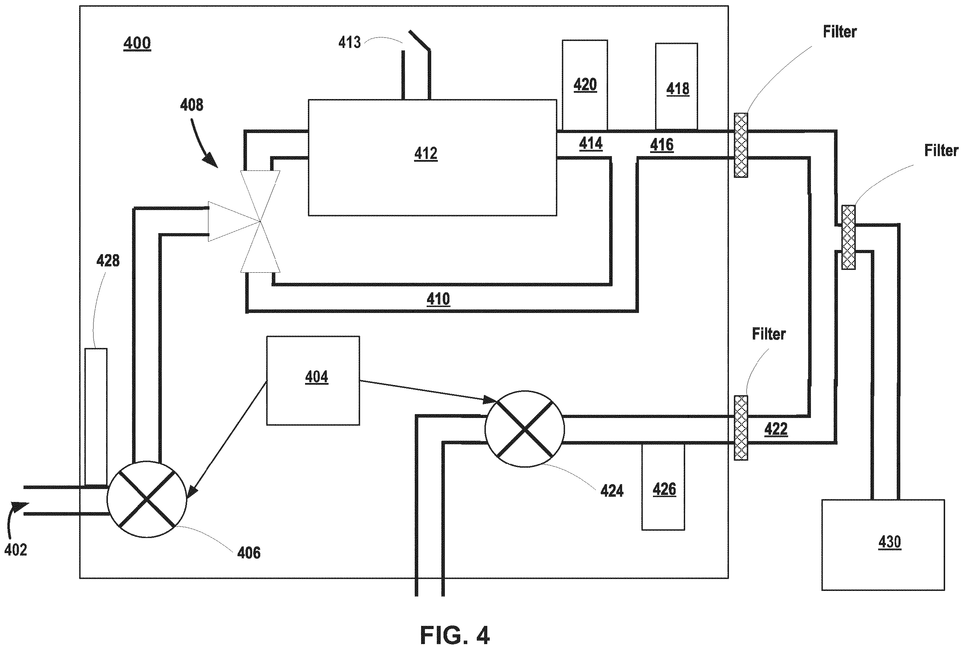

FIG. 4 is a conceptual diagram of a ventilator 400 including an example injector 312, in accordance with the techniques of this disclosure. Ventilator 400 includes pneumatic connection 402, pressure sensor 428, electronic control circuitry 404, inspiratory valve 406, 3-port valve 408, path 410, path 414, air inlet 413, injector 412, path 416, pressure sensor 418, dump valve 420, path 422, expiratory valve 424, pressure sensor 426. Ventilator 400 may be an example of ventilator 300 of FIG. 3. Although injector 412 and its operation is described in the context of ventilator 400 and supply gas, ambient air, and oxygen gas, injector 412 may be used independently or with any suitable system and may be used with other fluids such as other gases and/or gas/liquid mixtures.

A user, such as a doctor, nurse, or other medical clinician, may connect ventilator 400 to a gas supply via a pneumatic connection 402. Pneumatic connection 402 may, for example, include piping and/or tubing defining a pathway configured to guide supply gas from a gas supply into ventilator 400. This disclosure will explain the operation of ventilator 400 with reference to a gas supply that is 100% oxygen, but other gas and/or liquid/gas (e.g., fluid) supplies may also be used.

Electronic control circuitry 404 may include, or be coupled to, a user interface device that allows a user of ventilator 400 to set operating parameters for ventilator 400. Electronic control circuitry 404 may, for example, be a variable-frequency-variable-duty-cycle electronic control circuit that forms the basis of the control of ventilator 400. Electronic control circuitry 404 can provide the timing framework which is used to deliver conditioned gas flow to the lungs of a patient, e.g., lungs 430.

Electronic control circuitry 404 is configured to control the opening and closing of inspiratory valve 406 at a specific frequency to regulate the amount of conditioned gas being supplied to the patient and to control a "breaths per minute" being supplied to the patient. Generally speaking, a longer open interval for inspiratory valve 406 results in ventilator 400 delivering to a patient a larger volume of conditioned gas per breath, and more open intervals (e.g., more open intervals per minute) results in ventilator 400 delivering more breaths per minute. Electronic control circuity 404 may alter the amount of conditioned gas being supplied and the number of breaths per minute being supplied by ventilator 400 by varying the open-close pattern of inspiratory valve 406. The volume of conditioned gas being driven into the patient may be known and selected by a user.

Electronic control circuitry 404 may be configured to open inspiratory valve 406 for a period of time controlled by timing circuitry of electronic control circuitry 404 to achieve a desired inspiration volume. The time for which inspiratory valve 406 is held open may depend on inlet pressure of the gas supply, e.g., connected to pneumatic connection 402.

The supply gas that passes through inspiratory valve 406 is directed to 3-port valve 408. 3-port valve 408 includes a gas inlet for receiving supply gas via inspiratory valve 406 and includes two outlets. 3-port valve 408 is generally configured such that all of the supply gas being received by the inlet is directed to the first outlet or all of the supply gas being received by the inlet is directed to the second outlet. The first outlet and second outlet may, for example, deliver the supply gas to a respective path of two distinct paths. In FIG. 4, path 410 is the oxygen (100%) path, and path 414 is the air mix (50%) path. These percentages are approximate, and other mixtures, i.e., other percentages of oxygen, may also be used. Although FIG. 4 shows ventilator 400 as having a 3-port valve and two paths, the techniques of this disclosure can also be extended to an N-port valve that has N-1 output ports and N-1 paths, where N is a suitable integer.

A user of ventilator 400 may provide a user input to ventilator 400 to cause ventilator 400 to provide 100% oxygen to a patient, in which case 3-port valve 408 directs the supply gas through path 410. In other cases, a user of ventilator 400 may provide a user input to ventilator 400 to cause ventilator 400 to provide 50% oxygen (or any suitable percentage oxygen) to a patient, in which case 3-port valve 408 directs the supply gas through path 414.

Path 414 includes injector 412 and air inlet 413. Injector 412 may allow the high pressure gas flowing out of 3-port valve 408 to create a low static pressure that draws in air through air inlet 413, which may be a check valve. Thus, in path 414, the gas coming from the connected gas supply is mixed with ambient air, which in most common use scenarios produces conditioned gas that has less oxygen than the gas coming from the gas supply.

Regardless of whether 3-port valve 408 directs the supply gas to path 410 or 414, the gas ultimately is routed to the lungs of a patient by path 416 during respiration. Path 416 includes pressure sensor 418 to measure a pressure of the conditioned gas being delivered to the lungs of a patient, e.g., as described with reference to pressure sensor 314 of FIG. 3.

A user of ventilator 400 may set a maximum respiratory pressure that is appropriate for a particular patient, which control circuitry 404 can determine using any suitable technique. During an inspiration period, pressure sensor 418 may determine a pressure in path 416, and if a user adjustable pre-defined peak pressure target is reached, then electronic control circuitry 404 may cause inspiratory valve 406 to close, thus holding the pressure for the remainder of the inspiration time. Each time the peak pressure setting is hit, curtailing the inspired volume of conditioned gas from being delivered, ventilator 400 may produce a visible or audible warning, such as a flashing light or beeping, thus giving the user feedback that the desired volume of conditioned gas is not being reached.

Ventilator 400 also includes dump valve 420, which provides a mechanical backup pressure reduction mechanism in the event that the operation of pressure sensor 418, electronic control circuitry 404, and inspiratory valve 406 does not adequately reduce the pressure in path 416. Dump valve 420 may, for example, be a spring valve configured to open at a specific pressure. That specific pressure may be 15.75 in WG, but other valves that open at different pressures may also be used.

Injector 412 includes a mixing region fluidically connected to and supplied by both the supply gas from 3-port valve 408 and ambient air via air inlet 413. The mixing region may also be fluidically connected and configured to output to path 414. Injector 412 may include a choke, e.g., a venturi, between the mixing region and 3-port valve 408 which may be configured to increase the speed of the supply gas in the choke. Injector may also include a choke between the mixing region and air inlet 413 which may be configured to increase the speed of ambient air (drawn into injector 412 via air inlet 413) in the choke. Both the supply gas choke and the ambient air choke within injector 414 may output to the mixing region. In some examples, the supply gas flows at a speed that is at least the speed of sound within the supply path choke and may provide motive force for drawing in ambient air via air inlet 413 and the secondary flow path. In some examples, the ambient air may flow at a speed that is at least the speed of sound within the secondary path choke. In some examples, choking both the supply path and the secondary path enables the mass flow of both the supply gas and the ambient air to be insensitive to downstream pressure, e.g., pressure variation in path 414 and/or 416. In some examples, injector 412 may provide a mixing ratio that is insensitive to downstream pressure by virtue of providing mass flows of the supply gas and the ambient air that are insensitive to downstream pressure variations.

Both path 410 and injector 412 may provide a choked flow into path 416 via nozzles that are sufficiently small, such that a pressure ratio across the nozzles results in a choked flow through the nozzles. This choked flow may be achieved, for example, with a pressure ratio that may be 2 to 1, or 1.5 to 1, or 1.25 to 1, or any suitable pressure ratio between the pressure at three-port valve 408 and path 416. With paths 410 and 414 producing a choked flow, the output rate (e.g., volume per second) of paths 410 and 414 may be determined based on the pressure in path 416, which can be sensed by pressure sensor 418, and may stay constant even if a pressure in paths 410 or 414 or downstream from paths 410 or 414 changes. By using a choked flow through paths 410 and 412 to limit a flow into path 416, electronic control circuitry 404 can determine the output rate of conditioned gas being delivered to path 416 as a function of the pressure determined by pressure sensor 428 regardless of a pressure downstream from path 416.

From the output rate, electronic control circuitry 404 can determine a volume of conditioned gas being delivered to a patient for a respiratory cycle. To increase the volume of conditioned gas for a future respiratory cycle, electronic control circuitry 404 can increase the amount of time which inspiratory valve 406 remains open. To decrease the volume of conditioned gas for a future respiratory cycle, electronic control circuitry can decrease the amount of time which inspiratory valve 406 remains open.

Path 422 includes pressure sensor 426 configured to measure the pressure of a fluid (e.g., air) in path 422, which generally correlates to an expiratory pressure of the lungs of the patient. A minimum pressure at which expiration flow ceases is referred to as the PEEP. Electronic control circuitry 404 may be configured to control the opening and closing of expiratory valve 424 based on a measured pressure obtained from pressure sensor 426, such that a pressure within path 422 does not fall below a desired PEEP. The PEEP may, for example, be a value set by a user of ventilator 400.

During expiration, air is released from the lungs of a patient and out of ventilator 400 via path 422 by opening expiratory valve 424. Electronic control circuitry 404 may synchronize the opening and closing of inspiratory valve 406 with the opening and closing of expiratory valve 424, such that both are not simultaneously open. The PEEP represents a desired residual pressure in the lung and may be specified by a user of ventilator 400. Electronic control circuitry 404 may cause expiratory valve 424 to close when the pressure has decayed to the PEEP. Electronic control circuitry 404 may cause expiratory valve 424 to open multiple times to bring the pressure in path 422 to the desired PEEP level. If the PEEP level is reached before the end of an expiratory period, then electronic control circuitry 404 may hold expiratory valve 424 closed for the remaining time of the expiratory period.

In FIG. 4, inspiratory valve 406 and expiratory valve 424 may each be pneumatic solenoid valves. Inspiratory valve 406 and expiratory valve 424 may, for example, be low cost off the shelf open/shut direct acting solenoids rather than more expensive proportioning solenoids. That is, inspiratory valve 406 and expiratory valve 424 may be solenoids that are configured to transition between a fully closed state and a fully open state without maintaining any sort of persistent partially shut and partially open state.

In FIG. 4, paths 410, 414, 416, and 422, as well as other paths described herein, each represent pneumatic paths that may be formed by some combination of tubes, pipes, inlets, outlets, valves, connectors, regulators, filters, and other such components. Paths 410, 414, and 416 may be implemented as part of an assembly specifically designed to minimize flow resistance and noise.

Ventilator 400 may also be configured to sense a depression in the expiratory pressure and automatically trigger a new inspiratory cycle if the patient attempts to take an early breath. For example, pressure sensor 426 can be configured to detect a depression, by a specified amount below ambient pressure, in pressure in path 422. Based on the detected depression, electronic control circuitry 404 can determine that the patient is demanding a new inspiratory cycle, and based on this feedback, automatically trigger a new inspiratory cycle.

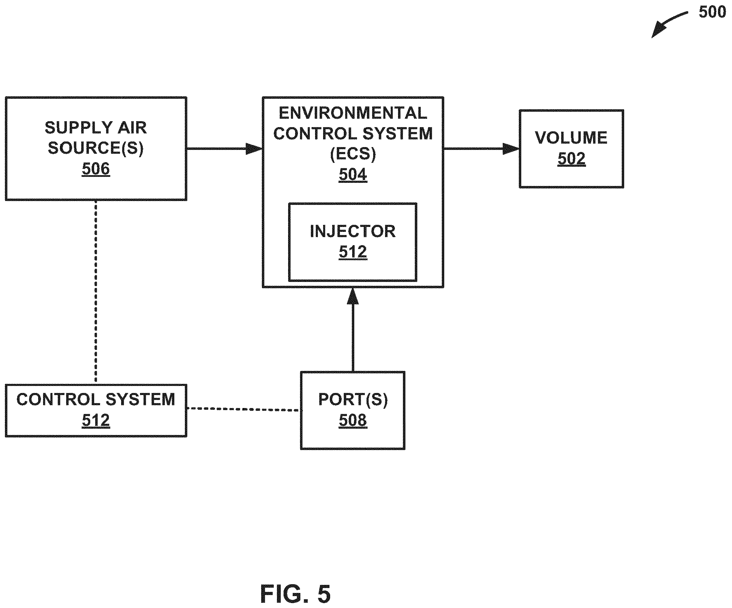

FIG. 5 is a conceptual diagram of an example system 500 including an example injector, in accordance with the techniques of this disclosure. Although system 500 is primarily be described with respect to an aircraft, it will be understood that the disclosed systems and techniques can be used for any other enclosed volume. For example, a watercraft, spacecraft, vehicle, a building, or other air-limited, controlled environment may utilize the disclosed systems and techniques for a constant and/or stable mass flow and mixing ratio of two fluids.

Volume 502 is configured to receive output, e.g., a mixed fluid, from environmental control system (ECS) 504. In some examples, volume 502 may be an aircraft cabin, a personnel space of a vehicle, a room in a building, or any other volume of space.

System 500 includes ECS 504. ECS 504 is configured to distribute conditioned air and/or fluid, e.g., an air-water mixture such as humidified air, into and throughout volume 502. For example, ECS 504 may receive pressurized air, such as bleed air from an engine or compressed air from a cabin air compressor, cool the pressurized air, such as using one or more heat exchangers and/or air conditioning packs, and distribute this conditioned air throughout cabin 502. ECS 504 may be configured to supply conditioned air to volume 502 under a variety of operating conditions, including grounded and/or docked conditions in which a cooling load is relatively high. To more evenly distribute air to personnel in volume 502, ECS 504 may be configured to distribute air throughout volume 502 in a relatively dispersed manner.

System 500 includes one or more supply air sources 506 (individually, "supply air source 506"). While shown as separate from ECS 504, in some instances, supply air source 506 may be part of ECS 504. Supply air source 506 is configured to discharge pressurized air into an air distribution system of ECS 504. System 500 also includes one or more ports 508 configured to provide fluidic access to ECS 504. For example, when an aircraft is at a grounded condition, ports 508 may provide access to supplemental conditioned air for supplying conditioned air to cabin 502.

In some examples, ECS 504 may be configured to supply a mixture of primary and secondary fluids, e.g., cool air mixed with hot air, water and/or water vapor mixed with an air flow path, and the like. ECS 504 may include injector 512. Injector 512 may include at least one choke, e.g., at least one venturi, supplied by supply air source 506, and at least one choke and/or venturi fluidically connected to a secondary flow path, e.g., supplied by ports 508. For example, one or more of ports 508 may be configured to be opened and closed to allow ambient air to mix with the supply air sources 506 within injector 512. In some examples, supply air from supply air sources 506 and the secondary flow path supplied ports 508 may include conduit, piping and/or tubing configured to define a pathway for a fluid, e.g., the supply gas and the secondary gas such as ambient air, respectively. A primary flow path from supply air sources 506 and the secondary flow path from ports 508 may be configured to allow a pressurized gas to communicate without leaking and may include one or more connectors and internal passage(s) to allow a gas to flow within the internal passage(s).

Injector 512 includes a mixing region fluidically connected to both supply air sources 506 and the secondary flow path after the chokes in both primary flow path and the secondary path. In some examples, the supply gas flows at a speed that is at least the speed of sound within the supply path choke and may provide motive force for drawing in ambient air via ports 508 and the secondary flow path. In some examples, the ambient air may flow at a speed that is at least the speed of sound within the secondary path choke. In some examples, choking both the supply path and the secondary path enables the mass flow of both the supply gas and the ambient air to be insensitive to downstream pressure, e.g., pressure variation in volume 502. In some examples, injector 512 may provide a mixing ratio that is insensitive to downstream pressure by virtue of providing mass flows of the supply gas and the ambient air that are insensitive to downstream pressure variations.

System 500 includes control system 512. Control system 512 may be configured to control one or more components of system 500 to provide conditioned air and/or fluid to volume 502. For example, control system 512 may be configured to control supply air sources 506 to discharge supply air into ECS 504 at a sufficient flow rate.

Ventilator 300, ventilator 400, and system 500 represent examples of devices and systems that may implement an injector configured in the manner described herein. It is contemplated that many other devices and systems could also implement such an injector. For example, an injector configured in the manner described herein, such as injectors 100 and/or 200, may be used as a pneumatic injector in any system and/or device using a pneumatic injector, e.g., a jet injector, an air gun, a jet pump, vacuum ejector, a vacuum pump, an aspirator, an air ejector, an air and/or air-liquid mixture injection system, a pneumatic injector, ejector, and/or pump, a heating, ventilating, and air conditioning (HVAC) system, an environmental control system, e.g., for a building, vehicle, or other volume of space, or any other system utilizing mixing at least two gases and/or gas-liquid mixtures.

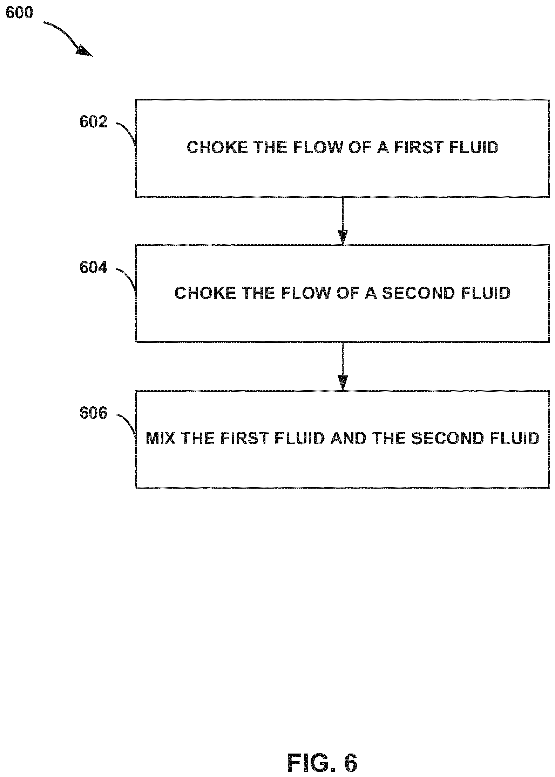

FIG. 6 is a flowchart of an example method 600 of mixing two fluids, in accordance with examples of the present disclosure. The method 600 below is described as being executed by and/or using injectors 100 or 200 of FIGS. 1-2, ventilators 300 or 400 of FIGS. 3-4, and ECS 504 of FIG. 5, however, the method 600 may be executed by any device or article suitable for using an injector and/or mixing fluids and/or entraining a first fluid within a second fluid.

Injector 100 may choke, via a first choke in a first flow path, the flow of a first fluid (602). For example, Injector 100 may include choke 104 configured to allow a constant mass flow of the first fluid to flow into a mixing region, e.g., mixing region 110, independent of a pressure at an outlet of the mixing region, e.g., outlet 112. For example, choke 104 may be configured such that pressure variations downstream of choke 104 have a reduced effect and/or no effect on the flow of the first fluid in the first flow path, e.g., primary flow path 102, and through choke 104.

Injector 100 may choke, via a second choke in a second flow path, the flow of a second fluid (604). For example, Injector 100 may include choke 108 configured to allow a constant mass flow of the second fluid to flow into the mixing region, e.g., mixing region 110, independent of a pressure at an outlet of the mixing region, e.g., outlet 112. For example, choke 108 may be configured such that pressure variations downstream of choke 108 have a reduced effect and/or no effect on the flow of the second fluid in the second flow path, e.g., secondary flow path 106, and through choke 108. In some examples, the second choke, e.g., choke 108, may be defined by a converging region upstream of the second choke and a diverging region downstream of the second choke, e.g., as illustrated in FIGS. 1-2.

Injector 100 may mix the first fluid and the second fluid in a mixing region (606). In some examples, the mixing region may be after the first choke of the first flow path and after the diverging region of the second flow path. For example, the first fluid may be a primary fluid, e.g., a driving fluid, of a jet pump and the second fluid may be a secondary fluid of the jet pump. The driving fluid may provide a motive force inducing the secondary fluid to flow from the secondary flow path into the mixing region, e.g., mixing region 110, where the secondary fluid may be mixed and/or entrained within the driving fluid.

In some examples the first choke and the second choke may be configured to cause a constant mixing ratio of the first fluid and the second fluid in the mixing region independent of the pressure at the outlet.

In some examples, choking the flow of the first fluid may include choking the flow of a first fluid such that the speed of the first fluid in the first choke is at least the speed of sound, wherein choking the flow of the second fluid may include choking the flow of a second fluid such that the speed of the second fluid in the second choke is at least the speed of sound.

In some examples, choking the flow of the second fluid may include choking, via a plurality of second chokes, the flow of the second fluid, wherein each of the plurality of second chokes may be defined by a converging region upstream of each second choke and a diverging region downstream of each second choke.

In some examples, each of the plurality of second chokes may be arranged in parallel in the second flow path. In some examples, each of the plurality of second chokes may be arranged in sequence in the second flow path. In some examples, one or more of the plurality of second chokes may be arranged in sequence and one or more of the plurality of second chokes may be arranged in parallel in the second flow path.

In some examples, the first fluid may be oxygen and the second fluid may be ambient air. In some examples, the first flow path and the second flow path may converge after the first choke and after the second choke.

Various examples have been described. These and other examples are within the scope of the following claims.

## Claims

1. An injector comprising:
a first conduit defining a first flow path for a first fluid, wherein the first flow path includes a first choke;
a second conduit defining a second flow path for a second fluid, wherein the second flow path includes a second choke, wherein the second choke is defined by a converging region upstream of the second choke and a diverging region downstream of the second choke;
a mixing region configured to receive the first fluid from the first flow path and the second fluid from the second flow path, wherein the mixing region is after the diverging region of the second flow path and after the first choke; and
an outlet configured to allow the first fluid and the second fluid to exit the mixing region,
wherein the first choke is configured to allow a constant mass flow of the first fluid to flow into the mixing region independent of a pressure at the outlet,
wherein the second choke is configured to allow a constant mass flow of the second fluid to flow into the mixing region independent of the pressure at the outlet.

2. The injector of claim 1, wherein the first choke and the second choke are configured to allow a constant mixing ratio of the first fluid and the second fluid in the mixing region independent of the pressure at the outlet.

3. The injector of claim 1, wherein the first choke is configured to cause a speed of the first fluid in the first choke to be at least the speed of sound, wherein the second choke is configured to cause a speed of the second fluid in the second choke to be at least the speed of sound..

4. The injector of claim 1, wherein the second flow path includes a plurality of second chokes, wherein each of the plurality of second chokes are defined by a converging region upstream of each second choke and a diverging region downstream of each second choke.

5. The injector of claim 4, wherein each of the plurality of second chokes are arranged in parallel in the second flow path.

6. The injector of claim 4, wherein each of the plurality of second chokes are arranged in sequence in the second flow path.

7. The injector of claim 4, wherein one or more of the plurality of chokes are arranged in sequence and one or more of the plurality of chokes are arranged in parallel in the second flow path.

8. The injector of claim 1, wherein the first fluid is oxygen and the second fluid is ambient air, wherein the first choke and the second choke are configured to supply a mixed mass flow of oxygen and ambient air such that a pressure ratio across the injector is less than or equal to 2.0, wherein the first flow path and the second flow path converge after the first choke and after the second choke.

9. A method of mixing a first fluid and a second fluid, the method comprising:
choking, via a first choke in a first flow path defined by a first conduit, the flow of a first fluid;
choking, via a second choke in a second flow path defined by a second conduit, the flow of a second fluid, wherein the second choke is defined by a converging region upstream of the second choke and a diverging region downstream of the second choke;
mixing the first fluid and the second fluid in a mixing region, wherein the mixing region is after the first choke of the first flow path and after the diverging region of the second flow path
wherein the first choke is configured to allow a constant mass flow of the first fluid to flow into the mixing region independent of a pressure at the outlet,
wherein the second choke is configured to allow a constant mass flow of the second fluid to flow into the mixing region for independent of the pressure at the outlet.

10. The method of claim 9, wherein the first choke and the second choke are configured to cause a constant mixing ratio of the first fluid and the second fluid in the mixing region independent of the pressure at the outlet.

11. The method of claim 9, wherein choking the flow of the first fluid comprises choking the flow of a first fluid such that the speed of the first fluid in the first choke is at least the speed of sound, wherein choking the flow of the second fluid comprises choking the flow of a second fluid such that the speed of the second fluid in the second choke is at least the speed of sound..

12. The method of claim 9, wherein choking the flow of the second fluid comprises choking, via a plurality of second chokes, the flow of the second fluid, wherein each of the plurality of second chokes are defined by a converging region upstream of each second choke and a diverging region downstream of each second choke.

13. The method of claim 12, wherein each of the plurality of second chokes are arranged in parallel in the second flow path.

14. The method of claim 12, wherein each of the plurality of second chokes are arranged in sequence in the second flow path.

15. The method of claim 12, wherein one or more of the plurality of second chokes are arranged in sequence and one or more of the plurality of second chokes are arranged in parallel in the second flow path, wherein the first fluid is oxygen and the second fluid is ambient air, wherein the first flow path and the second flow path converge after the first choke and after the second choke.
